# EUROPEAN PATENT APPLICATION

(11) **EP 1 066 794 A2**
(43) Date of publication of application: **10.01.2001**
(21) Application number: 00500078.1
(22) Date of filing: 24.04.2000
(51) Int. Cl.: A61B 6/00

(54) **Telescopic device applicable to the spatial positioning of x-ray outfits**

(30) Priority: 09.07.1999 ES 9901824; 10.02.2000 ES 200000302
(71) Applicant: Suinsa Subcontratas Internacionales, S.A., 28850 Torrejon de Ardoz (Madrid) (ES)
(72) Inventor: Ortega Jimenez, José Maria, "Las Monjas", 28850 Torrejon de Ardoz (ES); Rodenas Santiago, Juan, "Las Monjas", 28850 Torrejon de Ardoz (ES)
(74) Representative: Sanchez del Campo Gonzalez de Ubierna, Ramon

(57) **Abstract**

A telescopic device applicable to the spatial positioning of X-ray outfits, consisting of a frame (2), anchored at a roof, having an X-ray emitter (3) at its lower extremity, being displaced by gravity, and a braided cable (4), united with the lower extremity of the telescopic device (1) and a pulley (7) coaxially coupled to an electric motor (8), where the cable (4) is guided in its path by means of pulleys (7) and (7''), the pulley (7'') having a movable pulley support (5) coupled between two rails (10) and on which pneumatic springs (6) act with a chain device (9), pinions and an electric motor, incorporating a telescopic column guiding device (11).

## Description

### BACKGROUND OF THE INVENTION

The present specification relates to an application for a telescopic device applicable to the spatial positioning of X-ray outfits, the evident purpose of which is to be configured as an assembly of elements allowing the positioning of an X-ray outfit to be mechanically modified with regard to a definite source.

The invention enables an X-ray outfit to be placed on any datum point relative to the device source, in an acurate and fast way, electronically integrating all the - procedures derived from said positioning. In this way, the control of the driving motors of the positioning elements is wholly performed by means of electronically implemented methods, presenting devices detecting the positioning, which enable a great accuracy of motions.

The invention also contemplayes the guiding of telescopic columns applicable to medical apparatuses, such as X-ray outfits, providing an element consisting of five sections, which enables a proportional outlet and gathering of its constitutive parts, enabling a very smooth - guiding of if same.

### FIELD OF THE INVENTION

This invention will find application in the industry dedicated to the manufacture of elements, devices and systems applicable to medicine; in a most definite way, to the manufacture of X-ray outfits and devices related to their positioning.

### RELATED ART

It is known the existence of stationary X-ray oufits in which the positioning of the objective to be X-rayed is performed by moving said objective, adapting its position until being located within the acting field of the X-ray emitter.

It is also known the existence of X-ray outfits mounted on structures, through which they can rotate and move over rails or similar devices incorporated thetein. Optionally, the motion can be servomechanism - aided, enabling to carry out complex paths previously implemented by means of electronic and computerized devicas.

These X-ray outfits are, although allowing accurate paths free of human acts to be followed, scarcely suitable for being generally used, where it is imperative to rely on an ample margin of handling in order to can accede to any point within an ample space.

In this way, the above mentioned devices find application, for example, in dental surgery, where the whole field of application limits itself to a reduced volume.

Mechanical devices are known, which are formed starting from levers and counterweightd, optionally fitted with ball-and-socket joints, allowing an X-ray outfit to be placed on any point of the space, limited by the length of said lavers, enabling an ample margin of angle of incidence if it has ball-and-socket joints at the union node of the levers.

With these devices, it is not necessary to perform any efforts for modifying the position of the X-ray emitters, owing to the existence of counterweights. Nevertheless, accuracy is not remarkable, and involuntary displacements of the emitters are frequent.

It is also noted the need to move the objective to be X-rayed, passing through the path of complex evolutions, until reaching devices formed by levers and counterweights and fitted with ball-and-socket joints which, although eliminating motion efforts, lack an adequate accuracy.

On the lines of the above, it would be desirable, in view of the problems existing in this field, to rely on some device allowing an X-ray emitter to be placed on a datum point of any nature, relative to an arbitrary source, being also desirable that said device would present a high accuracy in said positioning, and would make it possible quick and, optionally, preprogrammed motions.

Nevertheless, the applicant is not aware of the existence at present of any device relying on the mentioned advantages.

### SUMMARY OF THE INVENTION

The telescopic device applicable to the spatial positioning of X-ray outfits as proposed by the invention, constitutes an evident novelty in its field of application, relying on the above mentioned advantages as desirable for a device of this type. Particularly, the invention allows an X-ray emitter to be situated on a datum point of any nature with regard to the device source and to carry out longitudinal motions in respect to - said source, placing the emitter in a point of relative coordinates Y, Z.

The invention is basically formed starting from a vertical descending telescopic device emerging from a base coupled to the roof of a room, at the lower end of same is an X-ray emitter.

The base is formed starting from a frame rigidly anchored to the roof presenting two longitudinal rails over which a movable pulley support displaces, from which a telescopic device hangs.

The vertical motion of the lower end of the telescopic device is performed by the action of an electric motor driving a pulley associated with a braided cable united, at its opposed end, with the lower end of the telescopic device, presenting guiding pulleys at intermediate points of its path.

In this way, the acting of the electric motor is translateted into a shortening or lengthening of the cable - lenght, reducing or increasing the datum of the telescopic device end with regard to its upper source.

The movable pulley support can move in a linear path along the guides by the constracting action of gas springs and an electric motor acting on said movable pulley support by means of a pinion and chain assenbly.

So, the action of the electric motor will generate an approach or withdrawal of the movable pulley support with regard to the source, the motor acting against the pneumatic springs, that is to say, the pneumatic springs always act in a sense, the position of the movable pulley support being controlled by the tension generated by the electric motor and its chain and pinion gear.

Obviously, the positioning at a different datum is performed by a relative sliding of the different sections of the telescopic element to its adjacent points, such as the conventional telescopic elements operate.

Also, in the invention it should be pointed out that, in a second embodiment, the outlet and gathering of the pipes can be effected in a proportional way, so affecting all the section at the same time.

Furthermore, as a consequence, the invention makes it possible to increase the smoothness of the guides, and, therefore, the pursued aim is to achieve an improved guiding of the sections constituting the column, this being an aspect emerging as the feature characterizing the key of the invention.

The invention relies on an X-ray emitter or similar at the lower extremity and emerging from a base installed under the roof of a room.

The telescopic element is formed by five hollow sections of cylindrical shape and diameters decreasing in vertical descending sense, and in order to produce the descent of the outfit located at its lower extremity, presents an aided servomotor acting on a pulley which causes the action of the braided cable united at its extremities with said pulley and the lower portion of the telescopic device.

Upon incorporating an aided servomotor, it is possible to reduce the effort of the operator in charge of the outfit, which becomes electronically driven.

The incorporation of the smooth guide device allows the ascending and descending vertical motion to be performed in all the sections at the same time, so guaranteeing a convenient smoothness to attain a level of adequate results.

### DESCRIPTION OF THE DRAWINGS

In order to complement this description and aid to a better understanding of the characteristics of the invention, the appending set of drawings, which is a part of this specification, shows, by way of illustrative and non-limiting example, the following:

Figure 1 shows an elevational view of the invention, showing the relative positioning of the telescopic device unfolded, the X-ray emitter at the lower extremity - thereof, and the frame anchored to the roof from which it emerges.

Figure 2 represents an isometric view of the invention related to a telescopic device applicable to the spatial positioning of X-ray outfits, showing also the arrangement of the elements integrating the interior of the frame.

Figure 3 represent an isometric view of the invention taken from different observation point.

Figure 4 shows a upper view of the frame, showing the arrangement of the elements housed, and the position of a vertical plane A-B generating the following figure.

Figure 5 corresponds to a view representing a sectional view generated by the plane A-B, the position of - which is detailed in figure 4.

Figure 6 represents a side view of the guiding device of the telescopic columns of the invention, showing the resultant picture when the assembly is folded.

Figure 7 represents a side view complementary of the preceding one, where the telescopic device shows its - constitutive zones spreaded.

Figure 8 illustrates a cross sectional view showing the arrangement of the guides, as well as an detail thereof.

Figure 9 shows a perspective view representing the manner in which the guiding of the sections of the column is performed.

### DESCRIPTION OF THE PREFERRED EMBODIMENT OF THE INVENTION

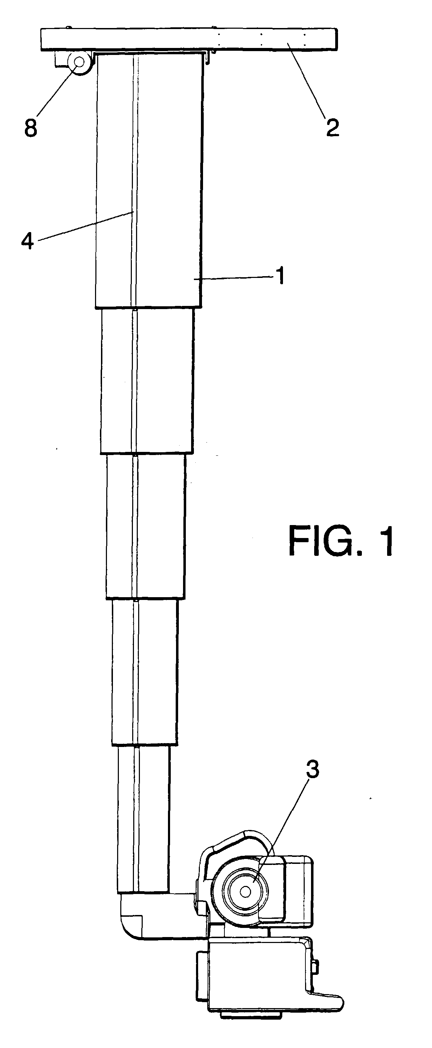

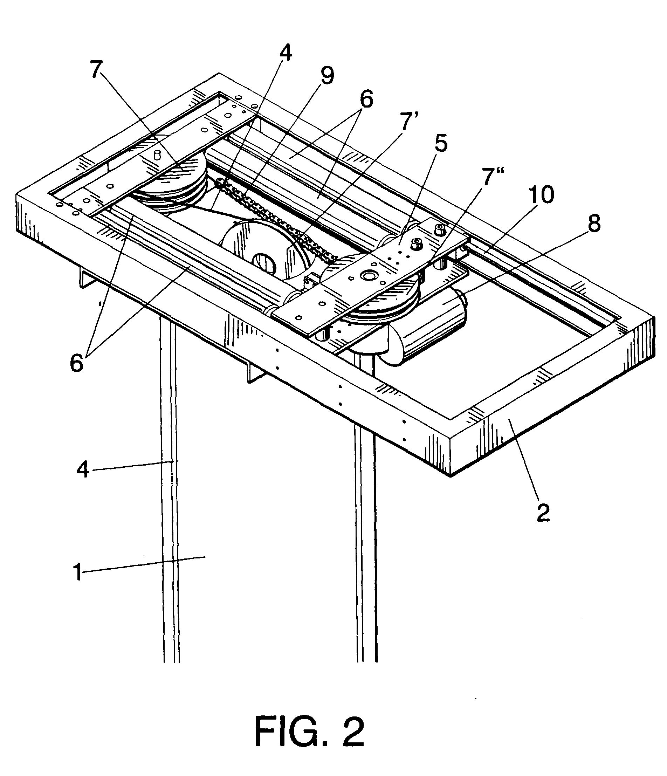

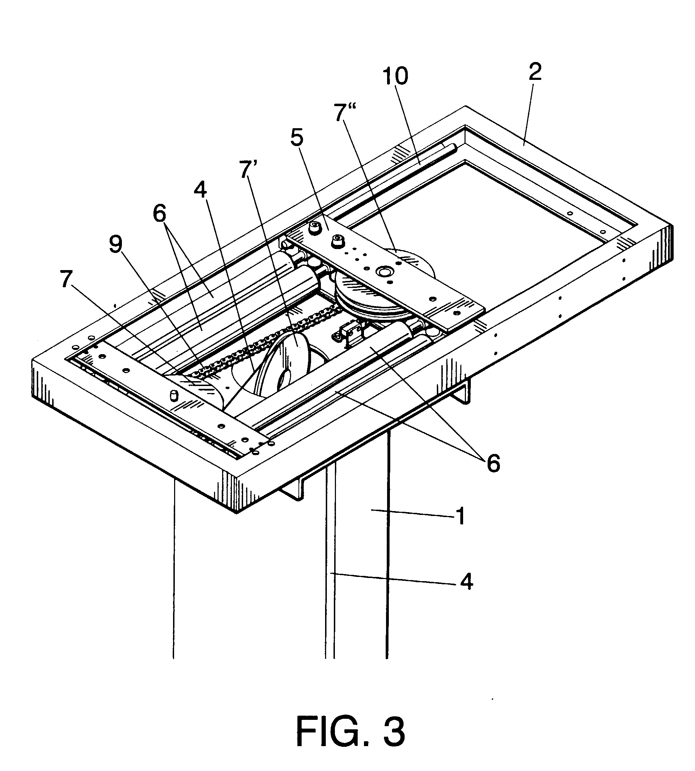

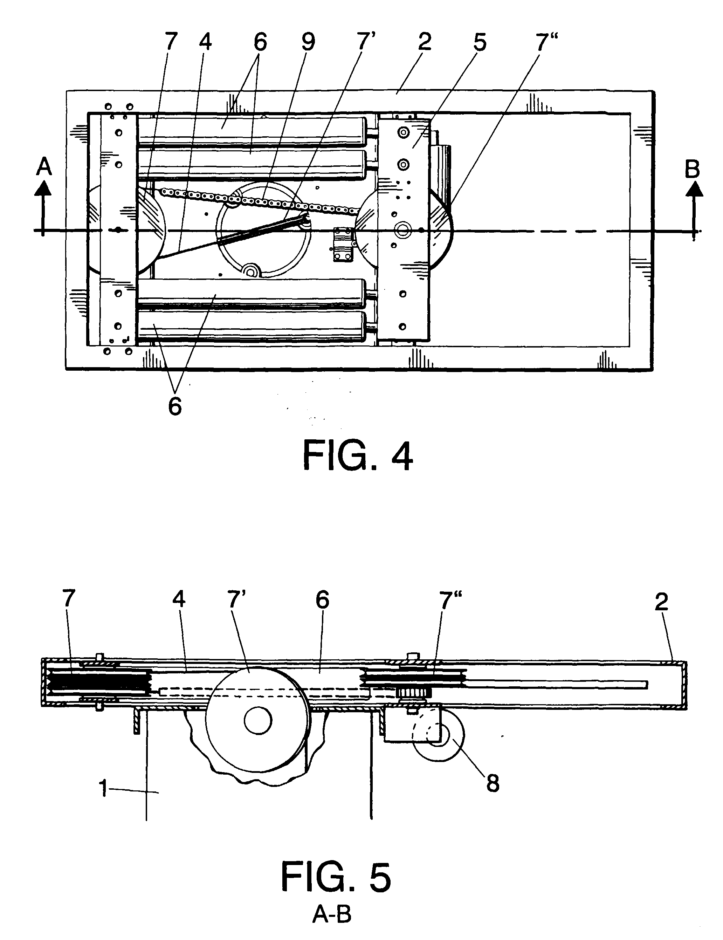

From figures 1, 2, 3, 4 and 5, it can be seen that the telescopic device applicable to the spatial positioning of X-ray outfits of the invention, is constituted starting from a telescopic device (1), vertically emerging from a frame (2), anchored at a roof, and supporting an X-ray emitter (3) at the lower end thereof, being moved by the joint action of gravity and a braided cable (4), united to the lower end of the telescopic device (1) and to a pulley (7) coaxially coupled to an electric motor (8).

The braided cable (4) is guided, in its path, by - means of pulleys (7) and (7"), the pulley (7") being located in a movable pulley support (5), coupled between two rails (10), and on which pneumatic springs (6) and a chain device (9), pinions and electric motor opposedly act, although it is optionally possible to replace the pneumatic springs (6) by hydraulic or pneumatic springs replacing the antagonistic action positioning the pneumatic springs (6) and the assembly made up by the chain (9), the pinions and the electric motor.

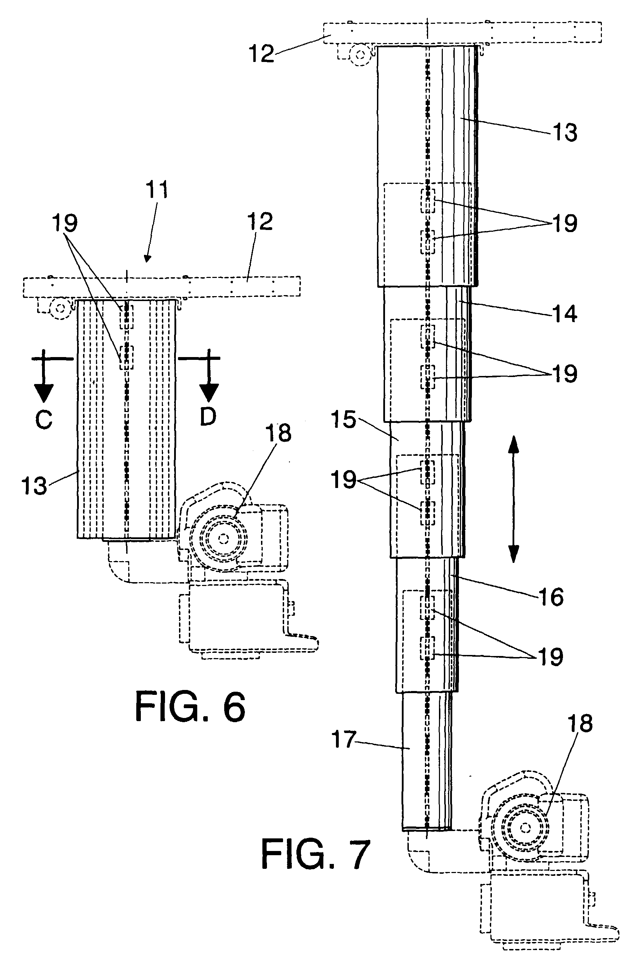

Following figures 6, 7, 8 and 9, it can be seen that the invention incorporates a telescopic column guiding device (11), which is constituted by a telescopic mechanism composed of five sections (13), (14), (15), (16) and (17).

It has two elements (12) and (13) at its ends, so that it emerges from a base (12) fixed to the roof of the room housing the resultant unit, and it has an X-ray emitter or similar (18) at its terminal side.

A column made up by parts (13), (14), (15), (16) and (17), adopting a hollow cylinder configuration, the radius of which decrease following the mentioned order, locates the terminal outfit (18) on a datum point selected by the user.

Placed on the position next to the base (12), there is a cylinder (13) of greater diameter and comprising the following element (14) which also incorporates a cylinder (15) located behind it and including a cylindrical part (16), so that the cylinder (16) contains, in its turn, an element (17), next to the outfit (18).

Each of bodies (13), (14), (15), (16) and (17) has a double set of guiding elements (19) and (20) incorporating stuck guides, this aspect being essential in the assembly of the invention.

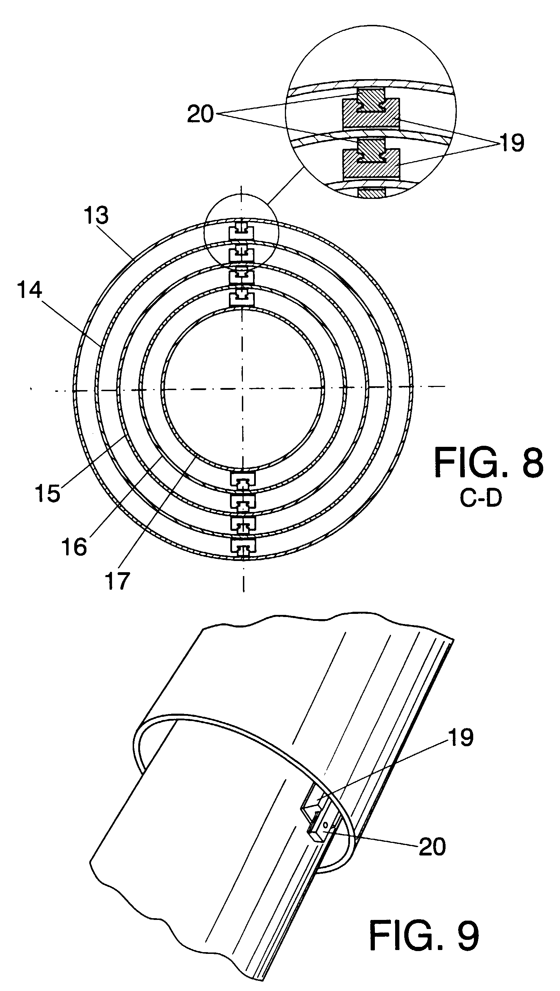

In order to assure that the guiding elements (19) and (20) fulfil well their functions, they are arranged occupying diametrically opposed positions, such as shown in figure 8.

The vertical ascending and descending motion is performed through the intervention of an aided servomotor causing a pulley to be driven, this pulley being associated with a braided cable which reduces or increases its length according to that imposed by the servomotor.

The inclusion of an aided servomotor enables the operator in charge of the outfit to reduce the necessary effort to be made, allowing, at the same time, the outfit to be electronically driven.

## Claims

1. A telescopic device applicable to the spatial positioning of X-ray outfits, characterized in that it is constituted starting from a telescopic device (1), emerging vertically from a frame (2), anchored at a roof, and having an X-ray emitter (3) at its lower extremity, being displaced by the joint action of gravity and a braided cable (4), united with the lower extremity of the telescopic device (1) and a pulley (7) coaxially coupled to an electric motor (8), where the braided cable (4) is guided in its path by means of pulleys (7) and (7"), the pulley (7") being located in a movable pulley support (5) coupled between two rails (10) and on which pneumatic springs (6) and a chain device (9) pinions and an electric motor opposedly act.

2. A telescopic device applicable to the spatial positioning of X-ray outfits, according to claim 1. characterized in that the pneumatic springs (6) can be replaced by hydraulic or pneumatic pistons replacing the antagonistic positioning action of the pneumatic springs (6) and the assembly made up by the chain (9), the pinion and the electric motor.

3. A telescopic device applicable to the spatial positioning of X-ray outfits, as claimed in claims 1 and 2, characterized in that it incorporates a telescopic column guiding device emerging from a base (12) anchored at a roof and having an X-ray emitter (18) or similar at its lower extremity, the telescopic column consisting of five sections (13), (14), (15), (16) and (17), adopting a configuration of hollow cylinders with decreasing diameters in vertical descending sense and coupled each others, sliding over a stuck guide device consisting of a double set of parts (19) and (20) occupying diametrically opposed positions in each of sections (13), (14), (15), (16) and (17), having an aided servomotor for the vertical ascending and descending motion.

4. A telescopic device applicable to the spatial positioning of X-ray outfits, according to claim 3, characterized in that the number of elements constituting the telescopic column guiding device can be variable.
